# EUROPEAN PATENT SPECIFICATION

(11) **EP 4 171 431 B1**
(45) Date of publication and mention of the grant of the patent: **30.07.2025**
(21) Application number: 20737850.6
(22) Date of filing: 24.06.2020
(51) Int. Cl.: A61B 90/00, A61B 17/00

(54) **BREAST BIOPSY MARKER CATHETER**
BRUSTBIOPSIEMARKERKATHETER
CATHÉTER MARQUEUR DE BIOPSIE DU SEIN

(43) Date of publication of application: 03.05.2023
(73) Proprietor: Bard Peripheral Vascular, Inc., Franklin Lakes, NJ 07417 (US)
(72) Inventor: KEIPER, Nicholas, Franklin Lakes, NJ 07417 (US); TEMPLE, Kyra, Franklin Lakes, NJ 07417 (US); WILLIS, Zachary, Franklin Lakes, NJ 07417 (US)
(74) Representative: Hoffmann Eitle
(86) International application number: PCT/US2020/039300
(87) International publication number: WO 2021/262157

(56) References cited:
- US-A1- 2005 192 616
- US-A1- 2016 100 843
- US-A1- 2016 228 126
- US-B1- 6 272 372

## Description

### Technical Field

The present invention relates to a breast biopsy apparatus, and, more particularly, to a breast biopsy marker and system.

### Background Art

Biopsy diagnostics and treatment often include the performing of a biopsy, such as a breast biopsy, in which a specimen or sample of tissue is removed from the patient at the biopsy site for pathological examination, tests and analysis. Obtaining a tissue sample by biopsy and the subsequent examination are typically employed in the diagnosis of cancers and other malignant tumors, or to confirm that a suspected lesion or tumor is not malignant. For example, a breast biopsy may be taken where a suspicious lump or swelling is noticed in a breast. Examination of tissue samples taken by the biopsy is of particular significance in the diagnosis and treatment of breast cancer.

After the biopsy is performed, it may take several days or weeks before the results of the examination of the sample are obtained, and still longer before an appropriate treatment decision is reached. If the decision involves surgery, it is important for the surgeon to find the location (biopsy site) in the breast from where the tumor tissue has been taken in the biopsy procedure, so that the entire tumor and possibly surrounding healthy tissue can be removed.

Various types of biopsy site markers are available, including visible markers applied externally to the patient's skin, radiographically (X-ray)-detectable tissue markers such as clips and staples, and ultrasound-detectable markers, to aid the physician in locating the biopsy site. However, such markers are not effective in filling the biopsy cavity for also providing a cosmetic benefit.

What is needed in the art is a breast biopsy marker and system that helps the physician in re-acquiring the location of the biopsy site, aids in patient healing, and/or provides a cosmetic benefit to the patient.
US 2005/0192616 A1 discloses a device for occluding a body lumen such that a reproductive lumen which includes an occluding component having an impervious barrier to provide initial occlusion of the body lumen and a permeable body to facilitate tissue ingrowth which provides long-term occlusion of the body lumen is disclosed.
US 2016/0100843 A1 discloses an apparatus for closure of an opening or isolation of a structure in a cardiovascular system. The apparatus includes an occluding segment, a delivery segment and a control segment.

### Summary of Invention

The present invention provides a breast biopsy marker and system that helps the physician in re-acquiring the location of the biopsy site, aids in patient healing, and/or provides a cosmetic benefit to the patient.

The presently claimed invention is defined in independent claim 1. Further developments of the herein claimed invention are described in the dependent claims.

An advantage of the present invention is that the breast biopsy marker helps the physician in re-acquiring the location of the biopsy site and provides a cosmetic benefit to the patient.

Another advantage of the present invention is that the breast biopsy marker may aid in healing by providing internal applied pressure at the biopsy site and a biodegradable scaffold / support for the tissue to heal around.

Another advantage of the present invention is that the breast biopsy marker may provide a cosmetic benefit to the patient by inflating the biopsy cavity.

Another advantage is that the breast biopsy marker is that it may be immediately placed and inflated following the biopsy through the existing biopsy tract, without the need for a new or future incision or puncture for marker placement.

Another advantage is that materials for the breast biopsy marker may be selected so as to provide both short term and long term imaging visibility in multiple imaging modalities.

### Brief Description of Drawings

The above-mentioned and other features and advantages of this invention, and the manner of attaining them, will become more apparent and the invention will be better understood by reference to the following description of an embodiments of the invention taken in conjunction with the accompanying drawings, wherein:
Fig. 1 is an exploded view of the breast biopsy marker system in accordance with an aspect of the present invention, with the bioabsorbable balloon of the marker catheter being in a deflated state;
Fig. 2 is a side view of the marker catheter of Fig. 1, with the bioabsorbable balloon being in an inflated state;
Fig. 3 is an enlarged section view of the marker catheter of Fig. 2, taken along line 3-3 of Fig. 2; and
Fig. 4 is a section view of the marker catheter of Fig. 3, taken along line 4-4-4-4 of Fig. 3.

Corresponding reference characters indicate corresponding parts throughout the several views. The exemplifications set out herein illustrate at least one embodiment of the invention, and such exemplifications are not to be construed as limiting the scope of the invention in any manner.

### Description of Embodiments

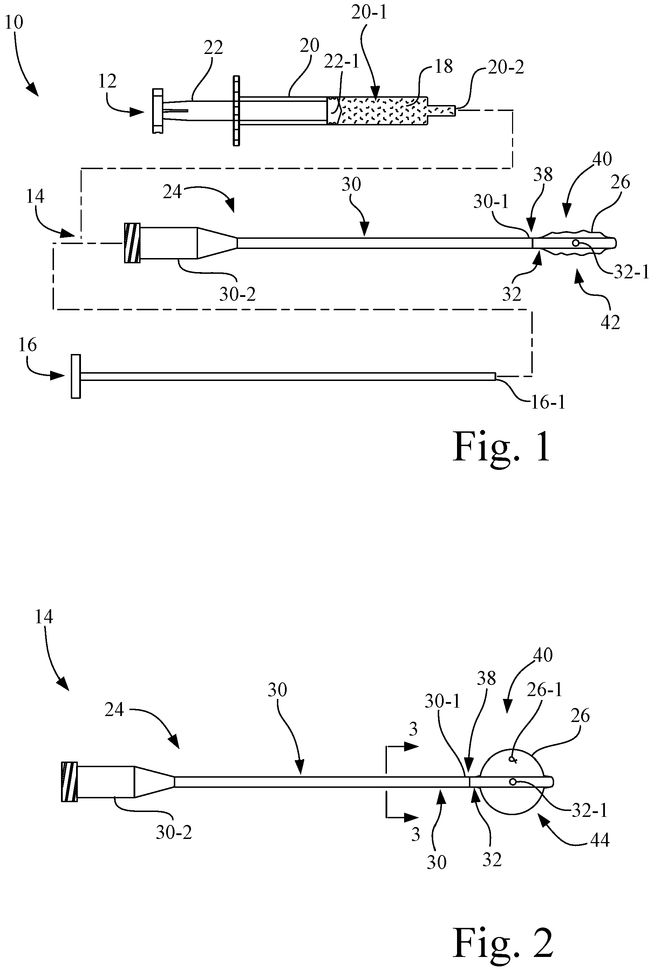

Referring now to the drawings, and more particularly to Fig. 1, there is shown a breast biopsy marker system 10 which generally includes a syringe 12, a marker catheter 14, and an elongate stylet 16.

Syringe 12 is configured to carry a gel material 18, such as a hydrogel. Syringe 12 may include, for example, a cylinder 20 having a chamber 20-1 and a plunger 22 having a piston 22-1 disposed in chamber 20-1 proximal to gel material 18. Cylinder 20 of syringe 12 has an injection port 20-2 that is connectable to marker catheter 14. Syringe 12 configured to deliver gel material 18 into marker catheter 14, as will be described in more detail below.

While in the present embodiment syringe 12 is shown as having a single chamber 20-1 that is configured to carry a pre-prepared gel as gel material 18, those skilled in the art will recognize that syringe 12 may be modified to have multiple (e.g., dual) chambers that contain the constituent component parts for forming a gel after the constituent component parts are combined. Accordingly, as used herein, the term "gel material" is intended to be interpreted broadly to include both pre-prepared gels and the constituent component parts which when combined, e.g., mixed, form a gel.

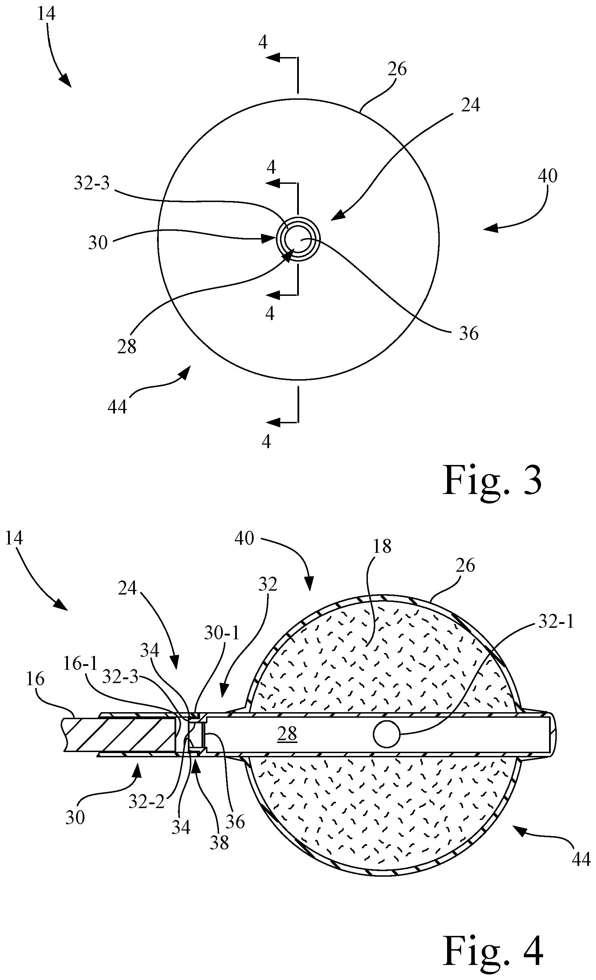

Referring also to Figs. 2 and 3, marker catheter 14 includes a catheter shaft 24 and a bioabsorbable balloon 26. Bioabsorbable balloon 26 may be made from a polymer, such as for example, a poly(glycolide-co-lactide) (PGLA) material, and optionally, may include at least one radiopaque and ultrasonically visible marker element 26-1, e.g., a titanium ribbon, that is imbedded in the PGLA material, so as to provide both short term and long term imaging visibility in multiple imaging modalities.

Catheter shaft 24 includes a lumen 28, a proximal tube portion 30, and a distal tube portion 32. Proximal tube portion 30 may be made, for example, of a non-biodegradable material. Distal tube portion 32 may be made, for example, of a non-biodegradable material and optionally may include a non-biodegradable component, e.g., etching, that provides permanent ultrasound visibility, thus yielding long term imaging visibility under ultrasound. The non-biodegradable material of proximal tube portion 30 may be, for example, a PEBAX^{®} brand polymer (PEBAX is a registered trademark of the Arkema France Corporation France). The non-biodegradable material and component of distal tube portion 32 may be, for example, polyvinyl alcohol.

In accordance with an aspect of the present invention, proximal tube portion 30 is joined to distal tube portion 32 by a frangible link 34. Distal tube portion 32 includes a one-way valve 36, e.g., a check valve, such as a pivotable flap, that is located in lumen 28, and a side wall orifice 32-1 in fluid communication with lumen 28. Injection port 20-2 of syringe 12 is releasably connectable, e.g., by a Luer or friction connection, to proximal tube portion 30 of catheter shaft 24 to facilitate delivery of gel material 18 into lumen 28 of catheter shaft 24.

In the present embodiment, with reference to Figs. 2-4, proximal tube portion 30 has a first end portion 30-1 and a hub portion 30-2, and distal tube portion 32 has a second end portion 32-2 having a proximal end surface 32-3. Second end portion 32-2 of distal tube portion 32 is positioned inside first end portion 30-1 of proximal tube portion 30, such that first end portion 30-1 of proximal tube portion 30 radially overlaps second end portion 32-2 of distal tube portion 32 to define an overlap region 38. As such, proximal end surface 32-3 of distal tube portion 32 is proximally exposed in lumen 28 of catheter shaft 24.

Frangible link 34 joins proximal tube portion 30 to distal tube portion 32 and may be located at overlap region 38 between proximal tube portion 30 and distal tube portion 32. For example, frangible link 34 may join proximal tube portion 30 to distal tube portion 32 by a breakable connection that is connected to each of proximal end surface 32-3 of second end portion 32-2 of distal tube portion 32 and proximal tube portion 30. In the present embodiment, for example, frangible link 34 is a spot weld.

Referring to Figs. 1-4, bioabsorbable balloon 26 is fixedly connected, e.g., via adhesive, laser welding, or shrink tubing, to distal tube portion 32 to define a balloon assembly 40. Syringe 12 is configured to deliver gel material 18 through lumen 28 of catheter shaft 24 to bioabsorbable balloon 26. Bioabsorbable balloon 26 has a deflated state 42 (see Fig. 1) and an inflated state 44 (see Figs. 2-4). In the deflated state 42, balloon assembly 40 of marker catheter 14 may be inserted into a biopsy cavity, e.g., a breast biopsy cavity, of a patient.

Referring particularly to Figs. 2-4, when injection port 20-2 of syringe 12 is connected to proximal tube portion 30 of catheter shaft 24, then bioabsorbable balloon 26 of balloon assembly 40 is coupled in fluid communication with syringe 12 via lumen 28 of catheter shaft 24. Accordingly, upon actuation of syringe 12, e.g., by depressing plunger 22, gel material 18 is delivered into bioabsorbable balloon 26 so as to inflate bioabsorbable balloon 26. In the present embodiment, bioabsorbable balloon 26 is configured for fluid communication with lumen 28 of catheter shaft 24 at a location distal to one-way valve 36 of distal tube portion 32 of catheter shaft 24. Accordingly, gel material 18 may be inserted into bioabsorbable balloon 26 through one-way valve 36 and side wall orifice 32-1 of distal tube portion 32 of catheter shaft 24 to effect the inflated state 44 of bioabsorbable balloon 26, wherein one-way valve 36 is configured to prevent a return from the inflated state 44 to the deflated state 42. In other words, as bioabsorbable balloon 26 of balloon assembly 40 is being inflated with gel material 18, then one-way valve 36 of distal tube portion 32 of catheter shaft 24 of balloon assembly 40 prevents a backflow of gel material 18, such that bioabsorbable balloon 26 is retained in a permanent inflated state 44.

Elongate stylet 16 is configured, e.g., as an elongate tube or rod, for insertion into lumen 28 of catheter shaft 24 to break frangible link 34 so as to separate proximal tube portion 30 of catheter shaft 24 from balloon assembly 40. In particular, elongate stylet 16 is configured for insertion into lumen 28 of catheter shaft 24 to break frangible link 34 of catheter shaft 24 following inflation of bioabsorbable balloon 26 with gel material 18 delivered by the syringe 12, so as to separate proximal tube portion 30 of catheter shaft 24 from balloon assembly 40.

For example, elongate stylet 16 is inserted into, and is advanced in, lumen 28 in proximal tube portion 30 of catheter shaft 24 such that distal end 16-1 of elongate stylet 16 engages proximal end surface 32-3 of second end portion 32-2 of distal tube portion 32 and/or frangible link 34. Elongate stylet 16 then may be further advanced, e.g., e.g., 0.5 to 2 millimeters, with a sufficient force so as to break frangible link 34, thereby separating proximal tube portion 30 from balloon assembly 40. After frangible link 34 is broken, then proximal tube portion 30 may be withdrawn from the patient while balloon assembly 40, in the inflated state 44, remains in the biopsy cavity of the patient.

Gel material 18 contained in bioabsorbable balloon 26 will be degraded in the patient over 12 to 18 months as bioabsorbable balloon 26 is resorbed. In some implementations, for example, gel material 18 may be, for example, a polyethylene glycol (PEG) based hydrogel, sodium hyaluronate (hyaluronic acid), or an aloe vera gel. Sodium hyaluronate encompasses wound-healing benefits and may be modified to exhibit thermoset properties. Thermoset properties of the gel material 18 may be desirable because it allows the material to be in a liquid state for inflation and then thicken to a gel-like consistency once it reaches body temperature. This allows for the ability to apply a minimal amount of pressure to inflate bioabsorbable balloon 26. In some implementations, PEG may be a desirable material due to its long-term, predictable degradation timeline and its ultrasound visibility properties. Aloe vera also encompasses wound-healing benefits, but does not allow for thermoset functionality.

## Claims

1. A breast biopsy marker catheter (14), comprising:
a catheter shaft (24) having a lumen (28), a proximal tube portion (30), and a distal tube portion (32), the proximal tube portion (30) being joined to the distal tube portion (32) by a frangible link (34), the distal tube portion having a one-way valve (36) located in the lumen (28); and
a bioabsorbable balloon (26) fixedly connected to the distal tube portion (32) to define a balloon assembly (40), the bioabsorbable balloon (26) configured for fluid communication with the lumen (28) of the catheter shaft (24) at a location distal to the one-way valve (36) of the distal tube portion (32) of the catheter shaft, the balloon assembly (40) configured to be separated from the proximal tube portion (30) of the catheter shaft by breaking the frangible link (34).

2. The breast biopsy marker catheter (14) according to claim 1, the bioabsorbable balloon (26) having a deflated state and an inflated state, and comprising a gel material (18) configured to be inserted into the bioabsorbable balloon (26) through the one-way valve (36) of the distal tube portion (32) of the catheter shaft to effect the inflated state of the bioabsorbable balloon (26).

3. The breast biopsy marker catheter (14) according to any one of claims 1 to 2, comprising:
the proximal tube portion (30) having a first end portion (30-1);
the distal tube portion (32) having a second end portion (32-2) having a proximal end surface (32-3), and
wherein the second end portion (32-2) of the distal tube portion (32) is positioned inside the first end portion (30-1) of the proximal tube portion (30) such that the first end portion (30-1) of the proximal tube portion (30) radially overlaps the second end portion (32-2) of the distal tube portion (32) to define an overlap region (38), and such that the proximal end surface (32-3) of the distal tube portion (32) is proximally exposed in the lumen (28) of the catheter shaft (24).

4. The breast biopsy marker catheter (14) according to claim 3, wherein the frangible link (34) that joins the proximal tube portion (30) to the distal tube portion (32) is located at the overlap region (38).

5. The breast biopsy marker catheter (14) according to claim 3, wherein the frangible link (38) that joins the proximal tube portion (30) to the distal tube portion (32) is connected to each of the proximal end surface (32-3) of the second end portion (32-2) of the distal tube portion (32) and the proximal tube portion (30).

6. The breast biopsy marker catheter (14) according to any one of claims 1 to 5, wherein the frangible link (34) is a spot weld.

7. The breast biopsy marker catheter (14) according to any one of claims 1 to 6, wherein the frangible link (34) is configured to break by using an elongate stylet (16) configured for insertion into the lumen (28) of the catheter shaft to break the frangible link (34) to separate the proximal tube portion (30) of the catheter shaft from the balloon assembly.

8. The breast biopsy marker catheter (14) according to any one of claims 1 to 7, wherein the bioabsorbable balloon (26) is made of a PGLA material, and further comprising at least one radiopaque and ultrasonically visible marker element imbedded in the PGLA material.

9. The breast biopsy marker catheter (14) according to any one of claims 1 to 8, wherein the proximal tube portion (30) is made with a non-biodegradable material and the distal tube portion (32) includes a non-biodegradable component configured to provide permanent ultrasound visibility.

10. The breast biopsy marker catheter (14) according to claim 9, wherein the non-biodegradable material of the proximal tube portion (30) is a PEBAX brand polymer and the non-biodegradable component of the distal tube portion (32) is polyvinyl alcohol.

11. A breast biopsy marker system (10), comprising:
a breast biopsy marker catheter (14) of any of the preceding claims,
a syringe (12) configured to carry a gel material (18), the syringe having an injection port (20-2) connectable to the proximal tube portion (30) of the catheter shaft (24), the syringe (12) configured to deliver the gel material (18) through the lumen of the catheter shaft to the bioabsorbable balloon (26); and
an elongate stylet (16) configured for insertion into the lumen (28) of the catheter shaft to break the frangible link (34) of the catheter shaft following inflation of the bioabsorbable balloon (26) with the gel material delivered by the syringe (12), so as to separate the proximal tube portion (30) of the catheter shaft from the balloon assembly (40).

## Patentansprüche

1. Brustbiopsiemarkerkatheter (14), umfassend:
einen Katheterschaft (24) mit einem Lumen (28), einem proximalen Rohrabschnitt (30) und einem distalen Rohrabschnitt (32), wobei der proximale Rohrabschnitt (30) mit dem distalen Rohrabschnitt (32) über eine zerbrechbare Verbindung (34) zusammengefügt ist, wobei der distale Rohrabschnitt ein Rückschlagventil (36) aufweist, das in dem Lumen (28) angeordnet ist; und
einen bioabsorbierbaren Ballon (26), der festsitzend mit dem distalen Rohrabschnitt (32) verbunden ist, um eine Ballonanordnung (40) zu definieren, wobei der bioabsorbierbare Ballon (26) für eine Fluidverbindung mit dem Lumen (28) des Katheterschafts (24) an einer zu dem Rückschlagventil (36) des distalen Rohrabschnitts (32) des Katheterschafts distalen Position konfiguriert ist, wobei die Ballonanordnung (40) dafür konfiguriert ist, von dem proximalen Rohrabschnitt (30) des Katheterschafts durch Zerbrechen der zerbrechbaren Verbindung (34) getrennt zu werden.

2. Brustbiopsiemarkerkatheter (14) nach Anspruch 1, wobei der bioabsorbierbare Ballon (26) einen entleerten Zustand und einen befüllten Zustand aufweist, und umfassend ein Gelmaterial (18), das zum Einleiten in den bioabsorbierbaren Ballon (26) durch das Rückschlagventil (36) des distalen Rohrabschnitts (32) des Katheterschafts konfiguriert ist, um den befüllten Zustand des bioabsorbierbaren Ballons (26) zu bewirken.

3. Brustbiopsiemarkerkatheter (14) nach einem der Ansprüche 1 bis 2, umfassend:
den proximalen Rohrabschnitt (30) mit einem ersten Endabschnitt (30-1);
den distalen Rohrabschnitt (32) mit einem zweiten Endabschnitt (32-2) mit einer proximalen Endfläche (32-3), und
wobei der zweite Endabschnitt (32-2) des distalen Rohrabschnitts (32) im Inneren des ersten Endabschnitts (30-1) des proximalen Rohrabschnitts (30) positioniert ist,
sodass der erste Endabschnitt (30-1) des proximalen Rohrabschnitts (30) den zweiten Endabschnitt (32-2) des distalen Rohrabschnitts (32) radial überlappt, um einen Überlappungsbereich (38) zu definieren, und sodass die proximale Endfläche (32-3) des distalen Rohrabschnitts (32) in dem Lumen (28) des Katheterschafts (24) proximal freigelegt ist.

4. Brustbiopsiemarkerkatheter (14) nach Anspruch 3, wobei die zerbrechbare Verbindung (34), die den proximalen Rohrabschnitt (30) mit dem distalen Rohrabschnitt (32) zusammenfügt, an dem Überlappungsbereich (38) angeordnet ist.

5. Brustbiopsiemarkerkatheter (14) nach Anspruch 3, wobei die zerbrechbare Verbindung (38), die den proximalen Rohrabschnitt (30) mit dem distalen Rohrabschnitt (32) zusammenfügt, mit jedem von der proximalen Endfläche (32-3) des zweiten Endabschnitts (32-2) des distalen Rohrabschnitts (32) und dem proximalen Rohrabschnitt (30) verbunden ist.

6. Brustbiopsiemarkerkatheter (14) nach einem der Ansprüche 1 bis 5, wobei die zerbrechbare Verbindung (34) eine Punktschweißung ist.

7. Brustbiopsiemarkerkatheter (14) nach einem der Ansprüche 1 bis 6, wobei die zerbrechbare Verbindung (34) dafür konfiguriert ist, unter Verwendung eines länglichen Stiletts (16), das zur Einführung in das Lumen (28) des Katheterschafts zum Zerbrechen der zerbrechbaren Verbindung (34) konfiguriert ist, zu zerbrechen, um den proximalen Rohrabschnitt (30) des Katheterschafts von der Ballonanordnung zu trennen.

8. Brustbiopsiemarkerkatheter (14) nach einem der Ansprüche 1 bis 7, wobei der bioabsorbierbare Ballon (26) aus einem PGLA-Material gefertigt ist, und weiter umfassend mindestens ein röntgendichtes und unter Ultraschall sichtbares Markerelement, das in dem PGLA-Material eingebettet ist.

9. Brustbiopsiemarkerkatheter (14) nach einem der Ansprüche 1 bis 8, wobei der proximale Rohrabschnitt (30) mit einem nicht biologisch abbaubaren Material gefertigt ist und der distale Rohrabschnitt (32) eine nicht biologisch abbaubare Komponente einschließt, die zum Bereitstellen einer permanenten Sichtbarkeit unter Ultraschall konfiguriert ist.

10. Brustbiopsiemarkerkatheter (14) nach Anspruch 9, wobei das nicht biologisch abbaubare Material des proximalen Rohrabschnitts (30) ein Polymer der Marke PEBAX ist und die nicht biologisch abbaubare Komponente des distalen Rohrabschnitts (32) Polyvinylalkohol ist.

11. Markersystem (10) für eine Brustbiopsie, umfassend:
einen Brustbiopsiemarkerkatheter (14) nach einem der vorstehenden Ansprüche,
eine Spritze (12), konfiguriert zum Tragen eines Gelmaterials (18), wobei die Spritze einen Injektionsport (20-2) aufweist, der mit dem proximalen Rohrabschnitt (30) des Katheterschafts (24) verbindbar ist, wobei die Spritze (12) zum Abgeben des Gelmaterials (18) durch das Lumen des Katheterschafts an den bioabsorbierbaren Ballon (26) konfiguriert ist; und
ein längliches Stilett (16), das zur Einführung in das Lumen (28) des Katheterschafts konfiguriert ist, um die zerbrechbare Verbindung (34) des Katheterschafts nach der Befüllung des bioabsorbierbaren Ballons (26) mit dem durch die Spritze (12) abgegebenen Gelmaterial zu zerbrechen, um den proximalen Rohrabschnitt (30) des Katheterschafts von der Ballonanordnung (40) zu trennen.

## Revendications

1. Cathéter à marqueur de biopsie du sein (14), comprenant :
un arbre de cathéter (24) présentant une lumière (28), une partie de tube proximale (30), et une partie de tube distale (32), la partie de tube proximale (30) étant reliée à la partie de tube distale (32) par une liaison frangible (34), la partie de tube distale présentant une valve unidirectionnelle (36) située dans la lumière (28) ; et
un ballonnet bioabsorbable (26) raccordé de manière fixe à la partie de tube distale (32) pour définir un ensemble ballonnet (40), le ballonnet bioabsorbable (26) étant configuré pour une communication fluidique avec la lumière (28) de l'arbre de cathéter (24) au niveau d'un emplacement distal de la valve unidirectionnelle (36) de la partie de tube distale (32) de l'arbre de cathéter, l'ensemble ballonnet (40) étant configuré pour être séparé de la partie de tube proximale (30) de l'arbre de cathéter par rupture de la liaison frangible (34).

2. Cathéter à marqueur de biopsie du sein (14) selon la revendication 1, le ballonnet bioabsorbable (26) présentant un état dégonflé et un état gonflé, et comprenant un matériau de gel (18) configuré pour être inséré dans le ballonnet bioabsorbable (26) à travers la valve unidirectionnelle (36) de la partie de tube distale (32) de l'arbre de cathéter pour donner effet à l'état gonflé du ballonnet bioabsorbable (26).

3. Cathéter à marqueur de biopsie du sein (14) selon l'une quelconque des revendications 1 ou 2, comprenant :
la partie de tube proximale (30) présentant une première partie d'extrémité (30-1) ;
la partie de tube distale (32) présentant une seconde partie d'extrémité (32-2) présentant une surface d'extrémité proximale (32-3), et
dans lequel la seconde partie d'extrémité (32-2) de la partie de tube distale (32) est positionnée à l'intérieur de la première partie d'extrémité (30-1) de la partie de tube proximale (30) de sorte que la première partie d'extrémité (30-1) de la partie de tube proximale (30) chevauche radialement la seconde partie d'extrémité (32-2) de la partie de tube distale (32) pour définir une région de chevauchement (38), et de sorte que la surface d'extrémité proximale (32-3) de la partie de tube distale (32) soit proximalement exposée dans la lumière (28) de l'arbre de cathéter (24).

4. Cathéter à marqueur de biopsie du sein (14) selon la revendication 3, dans lequel la liaison frangible (34) qui relie la partie de tube proximale (30) à la partie de tube distale (32) est située au niveau de la région de chevauchement (38).

5. Cathéter à marqueur de biopsie du sein (14) selon la revendication 3, dans lequel la liaison frangible (38) qui relie la partie de tube proximale (30) à la partie de tube distale (32) est raccordée à chacune de la surface d'extrémité proximale (32-3) de la seconde partie d'extrémité (32-2) de la partie de tube distale (32) et de la partie de tube proximale (30).

6. Cathéter à marqueur de biopsie du sein (14) selon l'une quelconque des revendications 1 à 5, dans lequel la liaison frangible (34) est une soudure par points.

7. Cathéter à marqueur de biopsie du sein (14) selon l'une quelconque des revendications 1 à 6, dans lequel la liaison frangible (34) est configurée pour se rompre par l'utilisation d'un stylet allongé (16) configuré pour être inséré dans la lumière (28) de l'arbre de cathéter pour rompre la liaison frangible (34) de manière à séparer la partie de tube proximale (30) de l'arbre de cathéter de l'ensemble ballonnet.

8. Cathéter à marqueur de biopsie du sein (14) selon l'une quelconque des revendications 1 à 7, dans lequel le ballonnet bioabsorbable (26) est fabriqué dans un matériau en PGLA, et comprenant en outre au moins un élément de marqueur radio-opaque et visible par ultrasons intégré dans le matériau en PGLA.

9. Cathéter à marqueur de biopsie du sein (14) selon l'une quelconque des revendications 1 à 8, dans lequel la partie de tube proximale (30) est fabriquée avec un matériau non biodégradable et la partie de tube distale (32) inclut un composant non biodégradable configuré pour fournir une visibilité ultrasonore permanente.

10. Cathéter à marqueur de biopsie du sein (14) selon la revendication 9, dans lequel le matériau non biodégradable de la partie de tube proximale (30) est un polymère de marque PEBAX et le composant non biodégradable de la partie de tube distale (32) est un alcool polyvinylique.

11. Système à marqueur de biopsie du sein (10), comprenant :
un cathéter à marqueur de biopsie du sein (14) selon l'une quelconque des revendications précédentes,
une seringue (12) configurée pour transporter un matériau de gel (18), la seringue présentant un orifice d'injection (20-2) raccordable à la partie de tube proximale (30) de l'arbre de cathéter (24), la seringue (12) étant configurée pour délivrer le matériau de gel (18) à travers la lumière de l'arbre de cathéter dans le ballonnet bioabsorbable (26) ; et
un stylet allongé (16) configuré pour être inséré dans la lumière (28) de l'arbre de cathéter pour rompre la liaison frangible (34) de l'arbre de cathéter suite à un gonflement du ballonnet bioabsorbable (26) avec le matériau de gel délivré par la seringue (12), de manière à séparer la partie de tube proximale (30) de l'arbre de cathéter de l'ensemble ballonnet (40).
